# FASCICULE DE BREVET EUROPEEN

(11) **EP 1 843 743 B1**
(45) Date de publication et mention de la délivrance du brevet: **31.10.2012**
(21) Numéro de dépôt: 06710217.8
(22) Date de dépôt: 04.01.2006
(51) Int. Cl.: A61K 9/00, A61J 7/00

(54) **BROYEUR ELECTRIQUE DE COMPRIME**
ELEKTRISCHE TABLETTENMUEHLE
ELECTRIC TABLET GRINDER

(30) Priorité: 07.01.2005 FR 0500188
(43) Date de publication de la demande: 17.10.2007
(73) Titulaire: CARDON PHARMACEUTICALS NV, 8200 Brugge (BE)
(72) Inventeur: CARDON, Chris, B-8200 Brugge (BE)
(74) Mandataire: Sabatier, Marc
(86) Numéro de dépôt international: PCT/IB2006/000003
(87) Numéro de publication internationale: WO 2006/072872

(56) Documents cités:
- EP-A- 0 855 183
- US-A- 5 067 666
- US-A- 5 148 995
- US-A- 6 059 209
- US-B1- 6 508 424
- US-B1- 6 637 683
- US-B2- 6 806 256
- "Tablet Grinder"[Online] 28 mai 2003 (2003-05-28), pages 1-3, XP002360776 Extrait de l'Internet: URL:http://www.halfbakery.com/idea/tablet_ 20grinder> [extrait le 2005-12-22]
- PATENT ABSTRACTS OF JAPAN vol. 2003, no. 06, 3 juin 2003 (2003-06-03) & JP 2003 053208 A (YUYAMA MANUFACTURING CO LTD), 25 février 2003 (2003-02-25)

## Description

La présente invention a pour objet un dispositif ou appareil pour la préparation d'une formulation pharmaceutique adaptée pour une administration par vole orale en terme de goût, de consistance et de dosage.

Les problèmes d'acceptation des médicaments (notamment sous forme de comprimés, tablettes, dragées, capsules, gélules ...) par voie orale chez les mammifères sont dans la plupart des cas dus au mauvais goût du médicament, à la taille du médicament qui est trop importante et aussi au fait que les gens âgés, les enfants ou les animaux de compagnie ont des difficultés à avaler le médicament.

La difficulté à avaler un médicament par voie orale pour les raisons évoquées ci-dessus est à l'origine du mauvais suivi par le patient d'un traitement prescrit par un médecin ou un vétérinaire, ce qui pose un sérieux problème. En effet, non seulement le patient ne guérit pas mais en outre l'arrêt du traitement peut aggraver sa situation.

Il existe également une limitation dans l'utilisation des comprimés mis sur le marché lorsque le dosage par comprimé n'est pas adapté aux espèces concernées, par exemple dans le cas des animaux de compagnie. Cet inconvénient est également gênant lorsque le dosage d'un médicament évolue en fonction de l'évolution d'une maladie.

Afin d'améliorer la tolérance d'un traitement, plusieurs solutions ont été décrites dans l'art antérieur. Ainsi, l'enrobage autour d'un comprimé, par exemple à base de sucre ou de résine, permet d'améliorer le goût d'un médicament. Cependant dans certains cas l'enrobage d'un comprimé ne permet d'obtenir que des comprimés de taille assez large,

Il est également possible d'effectuer uniquement l'enrobage autour des matières actives. Un tel enrobage sera par exemple à base de gomme ou de résine. De cette façon, on fabrique des granules ou un principe actif qui est prêt à être utilisé avec les autres ingrédients (excipients) pour faire un comprimé. L'inconvénient est cependant que l'on reste avec un enrobage spécifique au principe actif ; en outre la difficulté d'avaler facilement subsiste à cause de la taille du comprimé.

Pour certains médicaments, une formulation sous forme de sirop, de pâte ou de gouttes est disponible, ceci afin de trouver une solution pour administrer des médicaments comme le paracétamol, ta codéine, des vermifuges, l'halopéridol ... .

Malheureusement, la plupart des médicaments mis sur la marché ne sont disponibles que sous la forme de comprimés.

En général, lorsqu'un médicament ne peut pas être administré par la voie orale, il est alors administré sous forme d'injection ; l'inconvénient de cette méthode est que seul un professionnel de la santé peut l'administrer.

Il existe également une technique (dénommée « pill-popper ou pill-gun» en langue anglaise) dans laquelle on utilise une pince (du type pince à sucre) pour introduire directement dans la gorge le médicament (par exemple un comprimé entier). Cela comporte cependant la risque grave que le comprimé arrive dans les voles respiratoires au lieu des voies digestives.

Plusieurs documents de l'art antérieur concernent des dispositifs pour broyer des comprimés.

Le brevet US6508424 décrit un broyeur de comprimés portable fonctionnant avec des piles et comprenant un piston rotatif.

Le brevet US6806256 décrit un dispositif portable pour broyer des comprimés comprenant un vérin avec un ressort entraîné par une came.

Le document JP2003053208 décrit un broyeur pour comprimés qui se nettoie facilement en prévoyant sur le récipient de broyage une couche en composite fluoré, qui empêche l'adhésion de la poudre formée.

L'inventeur de la présente invention a maintenant mis au point un dispositif ou appareil permettant d'obtenir des formulations pharmaceutiques particulièrement bien adaptées pour une administration par voie orale, notamment en terme de consistance (gel, liquide, pâte ...) et de goût (agréable à la bouche).

Ainsi, la présente invention a pour objet un dispositif ou appareil destiné à la préparation d'une formulation pharmaceutique adaptée pour une administration par vole orale en terme de goût, de consistance et de dosage, obtenue selon un procédé qui comporte les étapes suivantes :
(a) broyage d'une formulation pharmaceutique solide, comprenant un ou plusieurs principes actifs et excipients, jusqu'à obtention d'une poudre et,
(b) mélange de la poudre ainsi obtenue avec :
   - un ou plusieurs excipients permettant de transformer ladite poudre en une formulation présentant une consistance adaptée pour une administration par voie orale (gel, liquide, pâte ...), et
   - un ou plusieurs excipients permettant de masquer le goût de la formulation pharmaceutique solide destinée à être broyée,
afin d'obtenir une formulation adaptée pour une administration par voie orale en terme de goût, de consistance et de dosage.

On entend par exemple par formulation pharmaceutique solide, un comprimé, une dragée, une tablette, une capsule, une gélule etc....,

Selon un mode de réalisation avantageux du procédé de l'invention, la poudre obtenue à l'issue de l'étape de broyage de la formulation pharmaceutique solide présente une granulométrie allant de 60 à 400 microns, et de préférence de 100 à 300 microns.

Selon un autre mode de réalisation avantageux du procédé de l'invention, le ou les excipients, permettent de transformer la poudre obtenue à l'issue de l'étape de broyage en une formulation présentant une consistance adaptée pour une administration par voie orale telle qu'un gel, un liquide ou une pâte sont choisis dans le groupe constitué par les résines, les polymères solubles ou non solubles dans l'eau.

Comme résine, on pourra par exemple utiliser l'Amberiite®, la Duolite®, l'Eudragit®.

A titre d'exemples de polymères solubles dans l'eau, on pourra citer l'hydroxypropylméthyl cellulose, l'hydroxypropyl cellulose, la polyvinylpyrrolidone et les alcools polyvinyliques.

A titre d'exemples de polymères non solubles dans l'eau, on pourra citer les copolymères méthacrylate et aminoalkyl méthacrylate tels que les copolymères éthyl acrylate/méthyl méthacrylate et les copolymères éthyl acrylate/méthyl méthacrylate/triméthylammonioéthyl méthacrylate. Ceux-ci sont vendus sous la marque Eudragit® (Röhm Pharma).

On entend par polymère non soluble dans l'eau, un polymère insoluble à une concentration de 10 mg par ml d'eau.

L'utilisation de polymères dans la préparation de la formulation pharmaceutique de l'invention permet d'obtenir une formulation présentant une viscosité allant de 50 mPa à 3000 mPa, et de préférence allant de 500 mPa à 1500 mPa.

La quantité d'excipients permettant de transformer la poudre obtenue à l'issue de l'étape de broyage en une formulation présentant une consistance adaptée pour une administration par voie orale, telle que par exemple une pâte, varie de 80 à 98% en poids, et de préférence de 90 à 96% en poids par rapport au poids total de la formulation orale finale.

On entend par formulation orale finale, la formulation obtenue à l'issue du procédé de préparation de l'invention.

Selon un autre mode de réalisation avantageux de l'invention, la ou les excipients permettant de masquer le goût de la formulation pharmaceutique solide destinée à être broyée sont choisis dans le groupe constitué par les exhausteurs de goût et les arômes.

En ajoutant un arôme ou un produit odoriférant, on obtient une formulation pharmaceutique particulièrement bien acceptée par les mammifères.

A titre d'exemple d'arôme on pourra citer un arôme dénommé « TCM F53 » (TCM Healthcare London Ltd, United Kingdom), du poulet, du poisson, du malt, du foie.

Les excipients qui sont responsables du masquage du goût parfois amer de certains médicaments sont par exemple des polymères qui forment un « réseau » autour du médicament en poudre, empêchant ainsi le contact direct du médicament avec les papilles gustatives sur la langue.

La quantité d'excipients permettant de masquer le goût de la formulation pharmaceutique solide destinée à être broyée varie de 4 % à 30 % en poids, et de préférence de 10 % à 20 % en poids par rapport au poids total de la formulation orale finale.

Selon un mode de réalisation avantageux de l'invention, il est également possible d'ajouter lors de l'étape de mélange, un ou plusieurs conservateurs tels que le sorbate de potassium, l'acide citrique, le benzoate de sodium, les sels d'édétate, un paraben tel que les p-hydroxybenzoates de méthyle, éthyle, propyle et butyle.

L'invention est encore caractérisée en ce que, lorsque la quantité de principe actif de la formulation pharmaceutique solide avant broyage varie de 1 % à 20 % en poids par rapport au poids total du comprimé, la quantité de principe actif de la formulation orale finale varie de 0,1 % à 5 % en poids par rapport au poids total de la formulation.

En principe, on ne peut pas administrer plus de cinq comprimés, car le masquage du goût devient alors difficile.

Le dispositif ou appareil destiné à la préparation d'une formulation pharmaceutique adaptée pour une administration par voie orale en terme de goût, de consistance et de dosage, comporte essentiellement :
- un moteur électrique (2),
- un broyeur (6) situé autour d'un axe (5),
- un mélangeur (7) situé autour de l'axe (5),
- un compartiment (8) permettant d'amener une formulation solide jusqu'au broyeur (6).

Le broyeur (6) est destiné à moudre une formulation pharmaceutique solide, par exemple un ou plusieurs comprimés, jusqu'à obtention d'un poudre présentant une granulométrie allant de 60 à 400 microns, et de préférence de 100 à 300 microns.

Le mélangeur (7) permet de mélangeur de manière homogène la poudre issue du broyage de la formulation orale avec le ou les excipients utilisés pour obtenir la consistance et le goût souhaités de la formulation pharmaceutique finale, jusqu'à obtention d'une formulation adaptée pour une administration par voie orale en terme de consistance (gel, liquide, pâte ...) et de goût.

Le dispositif de l'invention est plus particulièrement caractérisé en ce qu'il comporte en outre :
- une ou plusieurs piles (1),
- un ou plusieurs pignons (3) et (4),
- un ou plusieurs ressorts (9) à l'intérieur du compartiment (8),
- une ou plusieurs hélices (10) attachées à l'axe (5) situées à l'intérieur du mélangeur (7).

La moteur électrique (2) engendre la rotation des pignons (3) et (4), qui engendrent eux-mêmes la rotation de l'axe (5) et du broyeur (6) solidaire dudit axe (5). Le ou les ressorts (9) du compartiment (8) entraînent jusqu'au broyeur (6) une formulation solide destinée à être broyée.

Selon un mode de réalisation avantageux, le dispositif de l'invention est caractérisé en ce que le mélangeur (7) et le compartiment (8) sont des pièces amovibles du dispositif.

Selon un autre mode de réalisation avantageux, le dispositif de l'invention est caractérisé en ce qu'il comporte en outre une seringue graduée, pouvant être fermée.

Description des figures :
Les figures 1a et 1b représentent une vue en coupe du dispositif de l'invention.
Les figures 2, 3 et 4 représentent, en 3 dimensions, une vue détaillée de différents éléments constitutifs du dispositif de l'Invention.
Les figures 5a, 5b et 5c représentent, sous différents angles, une vue d'ensemble en 3 dimensions du dispositif de l'invention.
La figure 6 représente une vue de la seringue.

Les exemples suivants illustrent l'invention, ils ne la limitent en aucune façon.

### Exemple 1

### Formulation pharmaceutique sous forme liquide

On obtient 0,525 ml d'une poudre par broyage de 5 comprimés de 150 mg, ce qui équivaut à un total de 0,75 ml multiplié par le facteur de conversion moyen de 0,7).

| | |
|---|---|
| Poudre | 0,525 ml |
| Amberlite® 64 (résine polacrilex) | 10 % |
| Eudragit® EPO (copolymère méthacrylique) | 10 % |
| Propylèneglycol (humidifiant) | 10 % |
| Sirop de sorbitol à 70% (support) | 58 % |
| Benzoate de sodium (conservateur) | 1 % |
| Glycine (édulcorant) | 7 % |
| Goût (arôme de poulet) | 4 % |

A titre d'exemple de comprimé utilisé, on peut citer le Prednisolone 5 mg des laboratoires Kela.

### Exemple 2

### Formulation pharmaceutique sous forme de gel :

On obtient 0,7 ml d'une poudre par broyage de 5 comprimés de 200 mg, ce qui équivaut à un total de 1 ml multiplié par le facteur de conversion moyen de 0.7).

| | |
|---|---|
| Poudre | 0,7 ml |
| Carboxyméthylcellulose de sodium (épaississant) | 2 % |
| Propylèneglycol | 3 % |
| Sorbate de potassium (conservateur) | 1,2 % |
| Sirop de sorbitol à 70% | 68,8 % |
| Duolite® AP 143 (polymère styrène/divinylbenzène) | 5 % |
| NaOH tampon pH 9 | 17 % |
| Goût (arôme de poisson) | 3 % |

### Exemple 3

Préparation des excipients de la formulation décrite dans l'exemple 2 ci-dessus,

Les différents excipients qui seront mélangés à la poudre du comprimé sont préparés selon les quantités souhaitées et sont dans un premier temps mélangés entre eux dans l'ordre Indiqué ci-après.

Le carboxyméthylcellulose de sodium est humidifié par le propylèneglycol et l'on rajoute au mélange ainsi obtenu le Duolite AP 143.

L'arôme poisson est ensuite ajouté, puis le sorbate de potassium (conservateur).

Le sirop de sorbitol est progressivement ajouté, d'abord en petites quantités, puis de plus en plus, afin d'obtenir le volume nécessaire et qu'il soit homogène.

Le tampon est progressivement ajouté en petites quantités, jusqu'à ce que le pH soit d'une valeur de 9.

### Exemple 4

### Formulation Pharmaceutique sous forme de gel :

- Comprimés à broyer : 3,3 %
- Propylène Glycol : 10 %
- Methylcellulose de sodium : 2%
- Duolite® : 5 %
- Arôme de poisson : 5 %
- Sorbate de potassium : 1 %
- Sirop de sorbitol : 63, 7 %
- Tampon NaOH : 10%

### Exemple 5

### Mode de réalisation du dispositif de l'invention.

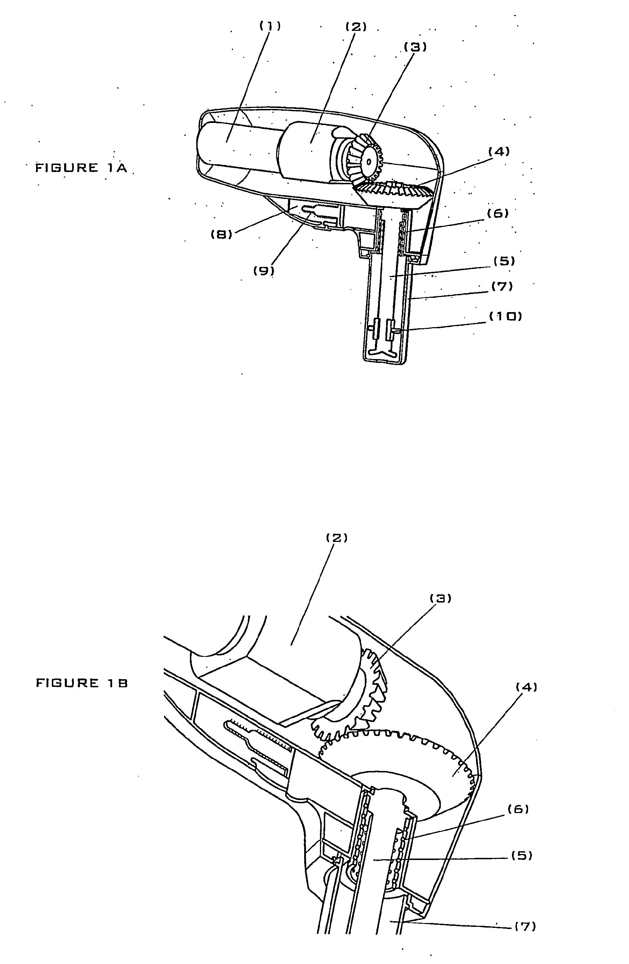

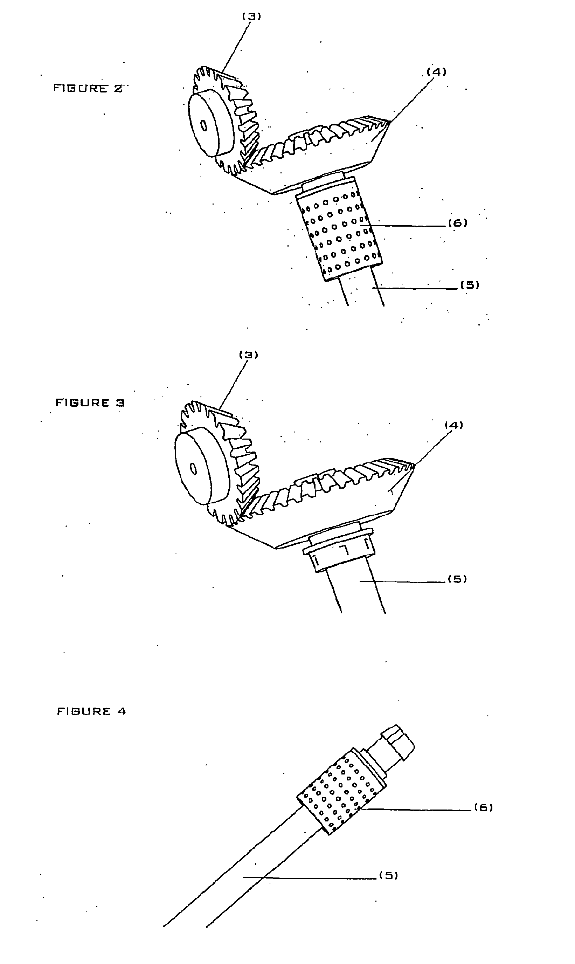

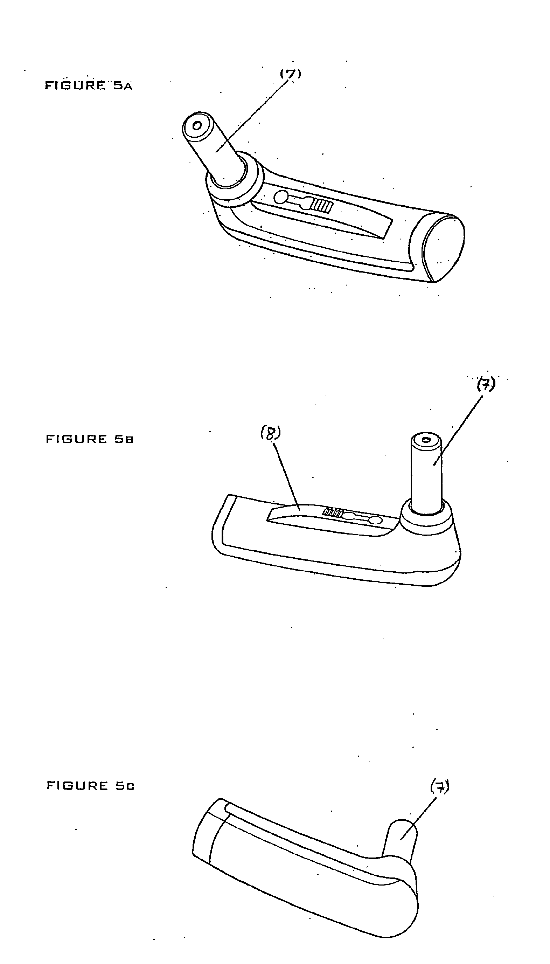

Le dispositif de l'invention, représenté schématiquement sur les figures 1 à 5, comporte plus particulièrement :
- une ou plusieurs piles (1),
- un moteur électrique (2) engendrant la rotation des pignons (3) et (4),
- un axe (5) entraîné par lesdits pignons (3) et (4), et entraînant la rotation du broyeur (6),
- un broyeur (6) destiné à broyer une formulation solide comme par exemple un comprimé, situé autour de l'axe (5),
- un compartiment (8) destiné à recevoir la formulation solide à broyer, comprenant un ou plusieurs ressorts (9) permettant d'amener ladite formulation jusqu'au broyeur (6),
- un mélangeur (7) situé autour de l'axe (5), muni d'une ou plusieurs hélices (10) attachées à l'axe (5).

Selon un mode de réalisation avantageux, il sera par exemple possible d'utiliser deux piles de forme cylindrique, de puissance 1,5 V. Les pignons (3) et (4), activés par le moteur électrique (2) présentent une rotation pouvant aller jusqu'à 5000 tours par minute. Lesdits pignons (3) et (4) sont réducteurs de vitesse, afin d'augmenter la puissance du moteur (2).

Selon un autre mode de réalisation avantageux, le broyeur (6) est de forme cylindrique avec un diamètre d'environ 1 cm et une épaisseur de 0,3 cm. Il est en aluminium inoxydable, tourne avec une vitesse maximum de 15000 rotations par minute et produit une poudre d'une granulométrie de 75 microns.

Le compartiment (8) est amovible afin d'en assurer l'entretien.

Le mélangeur (7) est destiné à mélanger respectivement :
(a) la poudre du comprimé broyé et,
(b) les excipients permettant de transformer ladite poudre en une formulation présentant un goût et une consistance adaptés pour une administration par voie orale.

Le mélangeur (7) comprend deux hélices (10) fixées sur l'axe (5) ; lorsque l'axe est en rotation, les hélices tournent dans des sens opposés.

Le mélangeur (7) est amovible, permettant ainsi de nettoyer le dispositif de l'invention et donc d'éviter une interaction avec d'autres comprimés précédemment broyés.

L'ensemble peut être monté dans une enveloppe étanche, un joint en caoutchouc ou en matière similaire assurant l'étanchéité entre la mélangeur 7 et les autres éléments.

Selon un autre mode de réalisation avantageux de l'invention, le dispositif comprend une seringue autonome ou reliée au mélangeur (7), par exemple une seringue graduée, permettant d'administrer l'ensemble des excipients au dosage voulu.

### Exemple 6

### Descriptif de la seringue

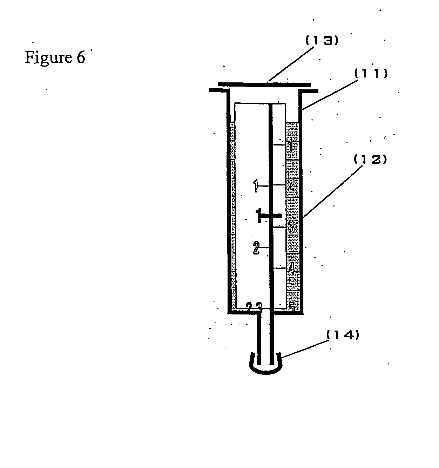

La seringue, représentée schématiquement sur la figure 6, comporte :
- un corps principal (11),
- une échelle de graduation (12),
- un couvercle étanche (13),
- un embout effilé et obturable (14).

Selon un mode de réalisation avantageux, le corps principal de la seringue aura un volume d'au moins 10 ml, une hauteur de 7 cm et un diamètre de 1,5 cm. Il sera en matériau transparent pour permettre de voir la réalisation du mélange. La seringue sera remplie avec 7,5 ml de la formulation. La moyenne du volume dégagé après mélange est de 8 ml. Ce volume varie en fonction du nombre de comprimés, du volume de chaque comprimé, du volume dégagé par l'agent actif, du volume dégagé par les excipients dans le comprimé.

Le corps principal de la seringue est muni d'une échelle, qui, selon un mode de réalisation, divise le volume total de la seringue en 5 parties, 4 parties. 3 parties ou 2 parties.

Le couvercle (13) est destiné notamment à permettre la conservation de la formule.

On peut apposer sur la seringue une étiquette pour noter le nom du patient, la date, le nom du médicament, le dosage, le nom du médecin ou du vétérinaire.

Pour utiliser la seringue en combinaison avec le dispositif mélangeur, après avoir enlevé le couvercle, on fixe ladite seringue sur le dispositif en la vissant, clipsant, ou emboîtant de tout autre manière équivalente.

Selon ce mode de réalisation, les comprimés sont broyés et la poudre est mixée dans le mélangeur avec les excipients pour obtenir une formulation sous forme d'une poudre qui tombe dans la seringue. La formulation est visible dans la seringue transparente, pour contrôle par l'opérateur, Il enlève ensuite la seringue et place le couvercle, il pose l'étiquette et met la seringue dans un sachet fermé jusqu'à utilisation de la formulation ainsi obtenue.

Selon un autre mode de réalisation, la seringue est utilisée seule à la fois comme moyen de mélange et de dosage. Ce cas d'utilisation est possible, lorsque le médicament n'a pas besoin d'être broyé. Après dosage, on procède comme indiqué ci-dessus.

## Revendications

1. Dispositif ou appareil destiné à la préparation d'une formulation pharmaceutique, adaptée pour une administration par voie orale en terme de goût, de consistance et de dosage et susceptible d'être obtenue selon un procédé comportant les étapes suivantes :
(a) broyage d'une formulation pharmaceutique solide, comprenant un ou plusieurs principes actifs et excipients, jusqu'à obtention d'une poudre et
(b) mélange de la poudre ainsi obtenue avec
• un ou plusieurs excipients permettant de former un gel, un liquide ou une pâte et
• un ou plusieurs excipients permettant de masquer le goût de ladite formulation pharmaceutique solide, **caractérisé en ce qu'**il comporte :
- un moteur électrique (2),
- un broyeur (6) situé autour d'un axe (5),
- un mélangeur (7) situé autour de l'axe (5),
- un compartiment (8) permettant d'amener une formulation solide jusqu'au broyeur (6).

2. Dispositif ou appareil selon la revendication 1, **caractérisé en ce qu'**il comporte en outre :
- une ou plusieurs piles (1),
- un ou plusieurs pignons (3) et (4),
- un ou plusieurs ressorts (9) à l'intérieur du compartiment (8),
- une ou plusieurs hélices (10) fixées à l'axe (5) et situées à l'intérieur du mélangeur (7).

3. Dispositif ou appareil selon la revendication 1 ou la revendication 2, **caractérisé en ce que** le moteur électrique (2) engendre la rotation des pignons (3) et (4), qui engendrent eux-mêmes la rotation de l'axe (5) et du broyeur (6) solidaire dudit axe (5).

4. Dispositif ou appareil selon l'une quelconque des revendications 1 à 3, **caractérisé en ce que** le ou les ressorts (9) du compartiment (8) amènent une formulation solide jusqu'au broyeur (6).

5. Dispositif ou appareil selon l'une quelconque des revendications 1 à 4, **caractérisé en ce que** le mélangeur (7) et le compartiment (8) sont des pièces amovibles.

6. Dispositif ou appareil selon l'une quelconque des revendication 1 à 5, **caractérisé en ce qu'**il comporte une seringue comprenant :
- un corps principal transparent,
- une échelle de volume,
- un couvercle amovible
- une sortie effilée et obturable.

7. Dispositif ou appareil selon l'une quelconque des revendication 1 à 6, **caractérisé en ce qu'**il est monté dans une enveloppe étanche et est muni d'un joint assurant l'étanchéité entre le mélangeur et les autres éléments.

## Claims

1. Device or apparatus for preparing a pharmaceutical formulation which is adapted for oral administration in terms of taste, consistency and dosage and can be obtained by a method comprising the following steps:
(a) grinding a solid pharmaceutical formulation, comprising one or more active principles and excipients, until a powder is obtained and
(b) mixing the powder thus obtained with
• one or more excipients for forming a gel, a liquid or a paste and
• one or more excipients for masking the taste of said solid pharmaceutical formulation, **characterised in that** it comprises:
- an electric motor (2),
- a grinder (6) situated about a shaft (5),
- a mixer (7) situated about the shaft (5),
- a compartment (8) for supplying a solid formulation to the grinder (6).

2. Device or apparatus according to claim 1, **characterised In that** it further comprises:
- one or more batteries (1),
- one or more pinions (3) and (4),
- one or more springs (9) inside the compartment (8),
- one or more impellers (10) fixed to the shaft (5) and situated Inside the mixer (7).

3. Device or apparatus according to either claim 1 or claim 2, **characterised in that** the electric motor (2) causes the rotation of the pinions (3) and (4), which themselves cause the rotation of the shaft (5) and of the grinder (6) which is rigidly connected to said shaft (5).

4. Device or apparatus according to any of claims 1 to 3, **characterised in that** the spring(s) (9) of the compartment (8) supply a solid formulation to the grinder (6).

5. Device or apparatus according to any of claims 1 to 4, **characterised in that** the mixer (7) and the compartment (6) are detachable parts.

6. Device or apparatus according to any of claims 1 to 5, **characterised in that** it comprises a syringe comprising:
- a transparent main body,
- a volume scale,
- a detachable cover
- a tapered and sealable outlet.

7. Device or apparatus according to any of claims 1 to 6, **characterised in that** it is mounted in a tight casing and is provided with a seal which ensures tightness between the mixer and the other elements.

## Patentansprüche

1. Vorrichtung oder Gerät für die Herstellung einer pharmazeutischnen Formulierung, die hinsichtlich Geschmack, Konsistenz und Dosierung für eine Verabreichung auf oralem Wege angepasst ist und die nach einem Verfahren erhältlich ist, das die folgenden Schritte umfasst:
(a) Zerkleinern einer festen pharmazeutischen Formulierung, die einen oder mehrere Wirkstoffe und Hilfsstoffe enthält, bis ein Pulver erhalten wird, und
(b) Mischen des auf diese Weise erhaltenen Pulvers mit
■ einem oder mehreren Hilfsstoffen, die die Bildung eines Gels, einer Flüssigkeit oder einer Paste ermöglichen;
■ einem oder mehreren Hilfsstoffen, die den Geschmack der festen pharmazeutischen Formulierung maskieren können;
**dadurch gekennzeichnet, dass** sie/es umfasst:
- einen Elektromotor (2);
- ein um eine Achse (5) angeordnetes Mahlwerk (6);
- einen um die Achse (5) angeordneten Mischer (7);
- eine Kammer (8), die es ermöglicht, eine feste Formulierung bis zu dem Mahlwerk (6) zu bringen.

2. Vorrichtung oder Gerät nach Anspruch 1, **dadurch gekennzeichnet, dass** sie/es ferner umfasst:
- eine oder mehrere Batterien (1),
- ein oder mehrere Getrieberäder (3) und (4),
- eine oder mehrere Federn (9) in Innern der Kammer (8),
- einen oder mehrere Flügel (10), die an der Achse (5) befestigt und im Innern des Mischers (7) gelegen sind.

3. Vorrichtung oder Gerät nach Anspruch 1 oder 2, **dadurch gekennzeichnet, dass** der Elektromotor (2) die Drehung der Getrieberäder (3) und (4) bewirkt, die wiederum die Rotation der Achse (5) und des fest mit der Achse (5) verbundenen Mahlwerks (6) bewirken.

4. Vorrichtung oder Gerät nach einem der Ansprüche 1 bis 3, **dadurch gekennzeichnet, dass** die Feder(n) (9) der Kammer (8) eine feste Formulierung bis zu dem Mahlwerk (6) bringen.

5. Vorrichtung oder Gerät nach einem der Ansprüche 1 bis 4, **dadurch gekennzeichnet, dass** der Mischer (7) und die Kammer (8) abnehmbar sind.

6. Vorrichtung oder Gerät nach einem der Ansprüche 1 bis 5, **dadurch gekennzeichnet, dass** sie/es ferner eine Spritze umfasst, die aufweist:
- einen transparenten Grundkörper,
- eine Volumenskala,
- einen abnehmbaren Deckel, und
- einen spitz zulaufenden und verschließbaren Auslass.

7. Vorrichtung oder Gerät nach einem der Ansprüche 1 bis 6, **dadurch gekennzeichnet, dass** sie/es in einem dichten Gehäuse eingebaut und mit einer Dichtung versehen ist, die die Dichtheit zwischen dem Mischer und anderen Elementen sicherstellt.
